Europäisches Patentamt

(19) European Patent Office

Office européen des brevets

(11) Numéro de publication : **0 039 289**
**B1**

(12) # FASCICULE DE BREVET EUROPÉEN

(45) Date de publication du fascicule du brevet :
**01.06.83**

(21) Numéro de dépôt : **81400651.6**

(22) Date de dépôt : **24.04.81**

(51) Int. Cl.³ : **C 07 C   7/04**, C 07 C   9/08

(54) **Procédé de fractionnement d'une charge d'hydrocarbures composée essentiellement d'hydrocarbures saturés ayant 2 à 4 atomes de carbone.**

(30) Priorité : **25.04.80 FR 8009326**

(43) Date de publication de la demande :
**04.11.81 Bulletin 81/44**

(45) Mention de la délivrance du brevet :
**01.06.83 Bulletin 83/22**

(84) Etats contractants désignés :
**BE DE FR GB IT NL**

(56) Documents cités :
**NL C 99 119**
**US A 2 577 617**

(73) Titulaire : **COMPAGNIE FRANCAISE DE RAFFINAGE**
**Société anonyme dite:**
**5, rue Michel-Ange**
**F-75781 Paris Cedex 16 (FR)**

(72) Inventeur : **Wiegandt, Herbert**
**Hampton Road 106**
**Ithaca New York (US)**

(74) Mandataire : **Jolly, Jean-Pierre et al**
**Cabinet BROT 83, rue d'Amsterdam**
**F-75008 Paris (FR)**

Procédé de fractionnement d'une charge d'hydrocarbures composée essentiellement
d'hydrocarbures saturés ayant 2 à 4 atomes de carbone

La présente invention concerne un procédé de fractionnement d'une charge d'hydrocarbures saturées composée essentiellement d'hydrocarbures saturés ayant de 2 à 4 atomes de carbone, à savoir l'éthane, le propane, le butane normal et/ou l'isobutane. Elle vise plus particulièrement à l'obtention d'une fraction riche en propane à partir de cette charge.

Dans un but de simplification, on entendra dans la suite de la présente description par le terme butane, soit du butane normal, soit de l'isobutane, soit un mélange de ces deux composés.

De nombreuses installations industrielles, telles que, par exemple, les raffineries de pétrole brut, possèdent des unités pour fractionner une charge d'hydrocarbures, composée essentiellement d'hydrocarbures saturés ayant de 2 à 4 atomes de carbone. Le fractionnement des hydrocarbures doit être effectué de la meilleure façon possible.

Dans un procédé connu, la charge composée essentiellement d'éthane, de propane et de butane est soumise à une première étape de distillation, conduisant à l'obtention :
— d'une part, d'éthane,
— d'autre part, d'un mélange de butane et de propane.

Le mélange de butane et de propane peut être utilisé tel quel. On procède généralement à une séparation par distillation du butane et du propane.

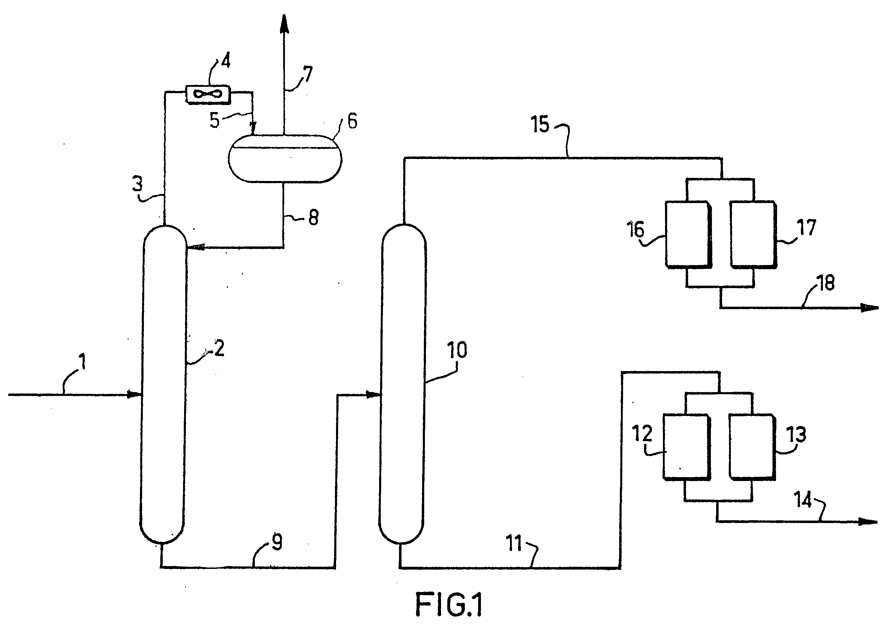

Une telle unité de fractionnement d'hydrocarbures saturés ayant de 2 à 4 atomes de carbone sera décrite immédiatement ci-après, pour plus de clarté, en référence à la figure 1 des trois dessins annexés.

Cette figure 1 est un schéma d'une unité d'un type connu pour le fractionnement d'hydrocarbures saturés ayant de 2 à 4 atomes de carbone ;

La figure 2 qui sera décrite ultérieurement, est un schéma d'un exemple de mise en œuvre du procédé selon l'invention ;

La figure 3 est un schéma d'une variante de cette mise en œuvre du procédé selon l'invention.

En référence à la figure 1, on introduit par la ligne 1, dans la partie médiane d'une colonne de distillation 2, appelée dééthaniseur, une coupe d'hydrocarbures composée essentiellement d'hydrocarbures saturés ayant de 2 à 4 atomes de carbone, mais pouvant toutefois contenir une quantité relativement faible d'autres hydrocarbures, comme du méthane par exemple. Cette coupe d'hydrocarbures peut être notamment obtenue par distillation sous pression atmosphérique d'un pétrole brut et séparation du méthane et des essences composées d'hydrocarbures contenant 5 ou plus de 5 atomes de carbone. Elle peut être également obtenue à partir d'une unité de reformage d'une essence.

Le dééthaniseur 2 fonctionne, par exemple, à une température qui peut être comprise entre 20 et 80 °C et à une pression qui peut être comprise entre 20 et 40 bars absolus.

Le fond du dééthaniseur est équipé d'un rebouilleur, qui, dans un but de simplification, n'a pas été représenté.

On recueille au sommet du dééthaniseur 2, par la ligne 3, la presque totalité de l'éthane contenu dans la charge de la ligne 1, ainsi qu'une certaine proportion de propane. Ce mélange est, après refroidissement dans un condenseur 4, par exemple un aéroréfrigérant, reconduit par la ligne 5 dans un ballon de reflux 6.

Le gaz est évacué de ce ballon de reflux 6 par la ligne 7, et le liquide est recyclé au sommet de la colonne 2 par la ligne 8. La ligne 7 est équipée d'un système, non représenté, permettant de régler la pression du gaz dans le ballon 6. La ligne 8 est équipée d'un système, non représenté, permettant de régler le niveau du liquide dans le ballon 6.

Le gaz évacué par la ligne 7, y compris le propane qu'il contient, est utilisé comme gaz combustible dans la raffinerie, mais il serait intéressant de pouvoir en séparer le propane pour d'autres applications.

On recueille au fond du dééthaniseur 2, par la ligne 9, une fraction d'hydrocarbures composée principalement de propane et de butane.

Il peut être envisagé d'utiliser cette fraction telle quelle, c'est-à-dire sans séparer le butane et le propane. En général, on effectue la séparation du butane et du propane.

A cet effet, la fraction contenue dans la ligne 9 est conduite dans la partie médiane d'une colonne de distillation 10, appelée dépropaniseur, fonctionnant, par exemple, à une température qui peut être comprise entre 20 et 80 °C et à une pression qui peut être comprise entre 10 et 30 bars absolus.

Le fond du dépropaniseur est équipé d'un rebouilleur, qui, dans un but de simplification, n'a pas été représenté.

On recueille au fond du dépropaniseur, par la ligne 11, un mélange constitué essentiellement de butane, qui est conduit sur une colonne de tamis moléculaire 12 ou 13, afin d'éliminer l'hydrogène sulfuré qu'il contient. L'une des colonnes est en service alors que l'autre est en régénération, grâce à un système de vannes non représenté. Le butane est recueilli par la ligne 14.

Le sommet du dépropaniseur est équipé d'un ballon de reflux, qui, dans un but de simplification, n'a pas été représenté.

On recueille au sommet du dépropaniseur, par la ligne 15, une fraction constituée essentiellement de propane, qui est conduite sur une colonne de tamis moléculaire 16 ou 17, afin d'éliminer l'hydrogène sulfuré qu'elle contient. L'une des colonnes est en service, alors que l'autre est en régénération, grâce à un système de vannes non représenté. Le propane est recueilli par la ligne 10.

Le procédé représenté sur la figure 1 présente donc l'inconvénient que le propane évacué par la ligne 7 avec l'éthane est mal utilisé et l'invention vise à proposer un procédé permettant de mieux séparer le propane de l'éthane.

Dans un but analogue, NL-C-99119 a déjà proposé un procédé de séparation par distillation d'une charge d'hydrocarbures en $C_1$ à $C_4$, conduisant à une fraction légère ($C_1$ et $C_2$) et une fraction plus lourde ($C_3$ et $C_4$), procédé dans lequel, en vue d'appauvrir la fraction légère en propane, on ajoute du butane aux produits légers en tête de colonne avant de les recycler dans la colonne principale de distillation.

La présente invention a pour objet un procédé de fractionnement d'une charge d'hydrocarbures composée essentiellement d'hydrocarbures saturés ayant de 2 à 4 atomes de carbone, en vue de l'obtention d'une fraction riche en propane, ledit procédé consistant à séparer par distillation ladite charge d'hydrocarbures en au moins deux fractions :

— une première fraction, A, comprenant la plus grande partie de l'éthane contenu dans ladite charge et une certaine quantité de propane, ladite première fraction étant soumise à un refroidissement conduisant à une phase liquide, qui est recyclée à ladite étape de distillation, et à une phase gazeuse, qui est évacuée,

— une deuxième fraction, B, comprenant la plus grande partie du butane contenu dans ladite charge et du propane en quantité plus importante que celle contenue dans la première fraction, ledit procédé étant caractérisé en ce que, avant la séparation de la phase liquide et de la phase gazeuse de ladite première fraction A, on procède à l'addition de butane à cette fraction, le butane ainsi additionné étant ensuite recyclé, avec ladite phase liquide, à ladite distillation, pour y être séparé, avec ladite deuxième fraction B, de la première fraction A.

Le procédé selon l'invention peut être notamment appliqué à l'obtention de propane et de butane, par séparation du mélange de butane et de propane.

La Demanderesse a en effet établi que l'addition de butane dans la première fraction permettait de réduire la quantité de propane contenue dans la phase gazeuse évacuée au cours de la première étape. Le propane est en effet absorbé par le butane.

La quantité de propane contenue dans la deuxième fraction B est plus importante, et le taux de récupération du propane est donc amélioré. La quantité de butane injectée peut être notamment comprise entre 0 et 200 % en poids de la quantité de propane contenue dans la charge d'hydrocarbures soumise à la distillation et, plus particulièrement, entre 0 et 50 % en poids de ladite quantité.

Ces pourcentages préférentiels sont notamment justifiés par des raisons économiques, qui seront expliquées plus loin.

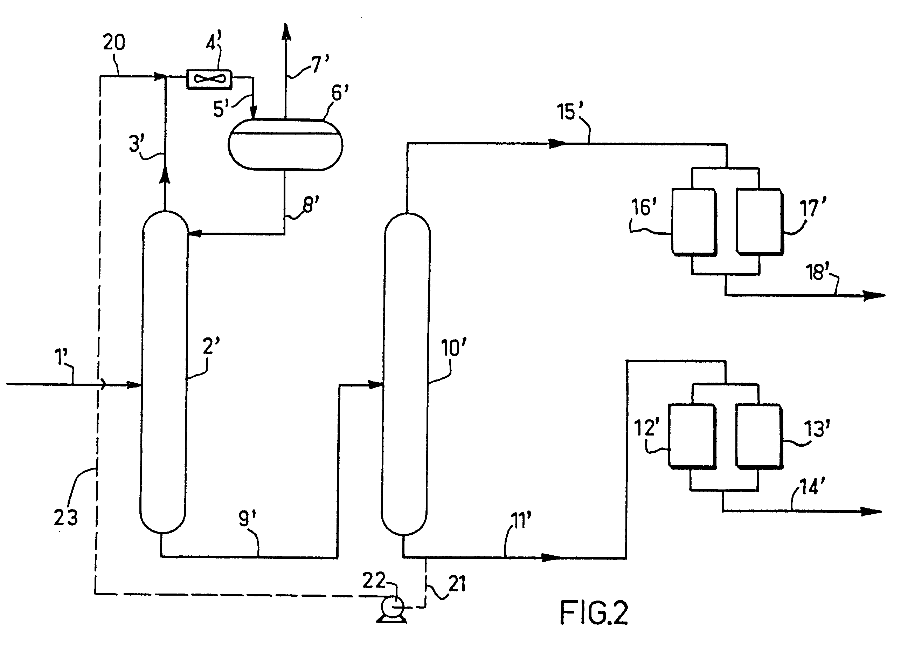

Un exemple d'application du procédé selon l'invention va maintenant être décrit, en référence à la figure 2 des dessins annexés, sur laquelle les éléments identiques à ceux de la figure 1 ont été désignés par les mêmes numéros de référence, affectés de l'indice '.

Dans cette application, le mélange de butane et de propane est soumis à une distillation, afin d'obtenir du butane et du propane.

Dans cette forme de mise en œuvre du procédé selon l'invention, l'injection de butane est effectuée par la ligne 20 dans la ligne 3', en amont de l'aéroréfrigérant 4'. Ce butane peut être notamment prélevé dans la ligne 11' par la ligne 21, repris par une pompe 22, d'où il est conduit à la ligne 20 par la ligne 23. Le butane peut également provenir d'un ballon de stockage situé en aval de la ligne 14' ou de toute autre source.

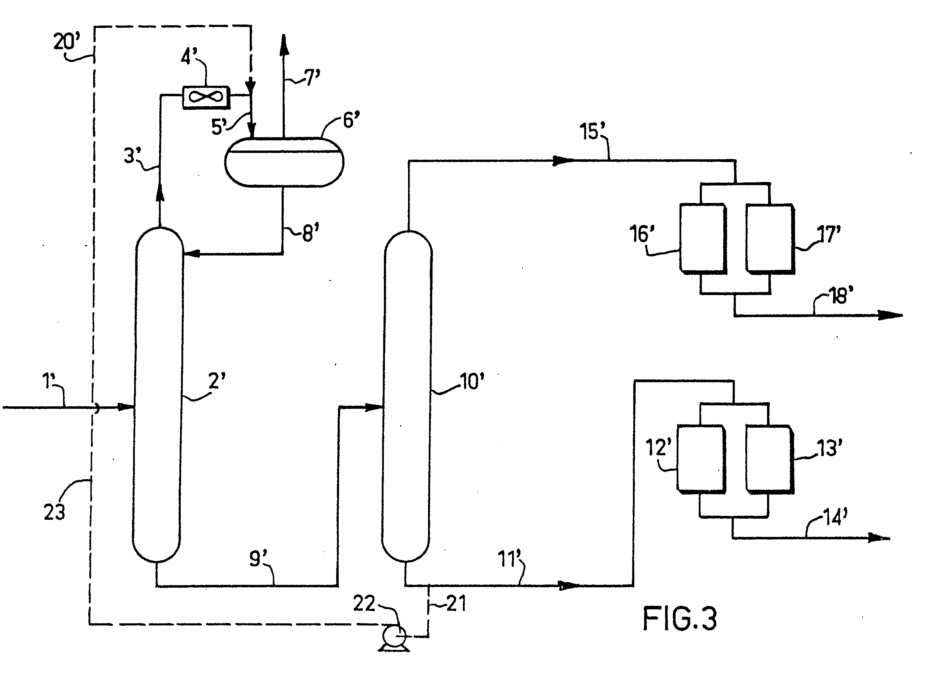

La figure 3 est un schéma d'une variante de mise en œuvre du procédé selon l'invention. Sur cette figure, les éléments déjà décrits en relation avec la figure 2 sont affectés des mêmes numéros de référence. Cette forme de mise en œuvre ne diffère de la procédure que pour ce qui concerne l'injection de butane, qui est effectuée par la ligne 20' dans la ligne 5', en aval de l'aéroréfrigérant 4', et non plus en amont de celui-ci.

L'invention est illustrée par l'exemple qui suit, qui n'a aucun caractère limitatif.

## Exemple

Quatre essais ont été effectués :

— un essai témoin T, conduit dans une installation telle que celle représentée sur la figure 1, c'est-à-dire sans injection de butane en tête du dééthaniseur ;

— des essais A, B et C, effectués dans une installation du type de celle représentée sur la figure 2, c'est-à-dire avec injection, par la ligne 20, de butane à des débits différents.

Les conditions opératoires des essais sont données dans le Tableau I ci-après.

(Voir tableaux 1 et 2, p. 4)

Tableau 1

| Références des figures | Conditions opératoires | Essai T | Essai A | Essai B | Essai C |
|---|---|---|---|---|---|
| Dééthaniseur 2 ou 2′ | Nombre de plateaux | 38 | 38 | 38 | 38 |
| | Température en tête (°C) | 52 | 44 | 29 | 20 |
| | Température en fond (°C) | 103 | 102 | 102 | 102 |
| | Pression (bars absolus) | 27 | 27 | 27 | 27 |
| Ligne 1 ou 1′ | Débit (t/h) | 25,56 | 25,56 | 25,56 | 25,56 |
| Ligne 20 | Débit (t/h) | 0 | 1,65 | 3,41 | 4,12 |
| Ligne 7 ou 7′ | Débit (t/h) | 3,93 | 2,84 | 2,27 | 2,12 |
| Ligne 9 ou 9′ | Débit (t/h) | 21,63 | 24,37 | 26,70 | 27,56 |

Lors de ces différents essais, on a analysé les compositions des lignes 1 ou 1′, 7 ou 7′, 9 ou 9′. Ces compositions sont données dans le Tableau 2 ci-après.

Dans le Tableau 3 qui suivra, on a indiqué également la quantité de propane contenue dans les lignes 7 ou 7′, c'est-à-dire la quantité de propane utilisée comme gaz combustible, en mélange avec l'éthane, et qui n'est donc pas valorisée.

Tableau 2

| | | | Composition en % poids | | | | | |
|---|---|---|---|---|---|---|---|---|
| Ligne n° | Essai | Hydrogène sulfuré | Ethane | Propane | Isobutane | Normal butane | Isopentane | Normal pentane |
| 1 ou 1′ | T, A, B ou C | 0,08 | 7,70 | 27,00 | 28,26 | 34,81 | 1,56 | 0,59 |
| 20 | T, A, B ou C | 0 | 0 | 0,43 | 44,13 | 55,44 | 0 | 0 |
| 7 ou 7′ | T | 0,51 | 50,12 | 49,34 | 0,03 | 0 | 0 | 0 |
| | A | 0,70 | 69,24 | 27,46 | 1,44 | 1,15 | 0 | 0 |
| | B | 0,88 | 86,64 | 8,86 | 1,98 | 1,64 | 0 | 0 |
| | C | 0,94 | 92,72 | 2,72 | 1,96 | 1,66 | 0 | 0 |
| 9 ou 9′ | T | 0,000 06 | traces | 22,95 | 33,98 | 41,13 | 1,85 | 0,69 |
| | A | 0,000 11 | traces | 25,15 | 32,46 | 40,13 | 1,64 | 0,62 |
| | B | 0,000 16 | traces | 25,15 | 32,52 | 40,27 | 1,50 | 0,56 |
| | C | 0,000 22 | traces | 24,90 | 32,66 | 40,45 | 1,45 | 0,54 |

4

Tableau 3

| Essai | Débit de propane (kg/h) dans la ligne 7 ou 7' |
|---|---|
| T | 1 938 |
| A | 781 |
| B | 201 |
| C | 58 |

Ces tableaux permettent de constater que la quantité de propane contenue dans la ligne 7' est de moins en moins importante au fur et à mesure que la quantité de butane injectée par la ligne 20 augmente.

Ce propane sera récupéré après le dépropaniseur 10' par la ligne 18'. Il pourra être valorisé comme propane commercial au lieu d'être utilisé comme gaz combustible.

L'injection de butane est limitée d'une façon générale par un facteur économique. En effet, il faut fournir à la tour 2 ou 2' de plus en plus de calories par son rebouilleur, au fur et à mesure que l'on injecte du butane.

## Revendications

1. Procédé de fractionnement d'une charge d'hydrocarbures composée essentiellement d'hydrocarbures saturés ayant 2 à 4 atomes de carbone en vue de l'obtention d'une fraction riche en propane, ledit procédé consistant à séparer par distillation ladite charge d'hydrocarbures en au moins deux fractions :
— une première fraction, A, comprenant la plus grande partie de l'éthane contenu dans ladite charge et une certaine quantité de propane, ladite première fraction étant soumise à un refroidissement conduisant à une phase liquide, recyclée à ladite étape de distillation, et à une phase gazeuse, qui est évacuée,
— une deuxième fraction, B, comprenant la plus grande partie du butane contenu dans ladite charge et du propane en quantité plus importante que celle contenue dans la première fraction, ledit procédé étant caractérisé en ce que, avant la séparation de la phase liquide et de la phase gazeuse de ladite première fraction A, on procède à l'addition de butane à cette fraction, le butane ainsi additionné étant ensuite recyclé, avec ladite phase liquide, à ladite distillation, pour y être séparé, avec ladite deuxième fraction B, de la première fraction A.

2. Procédé selon la revendication 1, caractérisé en ce que le butane est injecté en une quantité pouvant représenter jusqu'à 200 % en poids du propane contenu dans la charge d'hydrocarbures et, de préférence, jusqu'à 50 % en poids.

3. Application du procédé selon l'une des revendications 1 et 2 à l'obtention de propane et de butane.

## Claims

1. A process for fractionating a hydrocarbon charge formed essentially of saturated hydrocarbons having from 2 to 4 carbon atoms with a view to obtaining a propane rich fraction, said process consisting in separating by distillation said hydrocarbon charge into at least two fractions :
— a first fraction A comprising the largest part of the ethane contained in said charge and a certain amount of propane, said first fraction being subjected to cooling leading to a liquid phase, recycled to said distillation step, and to a gaseous phase, which is discharged,
— a second fraction B comprising the largest part of the butane contained in said charge and propane in an amount greater than that contained in the first fraction, said process being characterized in that, before separation of the liquid phase and the gaseous phase of said first fraction A, butane is added to this fraction, the butane thus added then being recycled, with the liquid phase, to said distillation, to be there separated, with said second fraction B, from the first fraction A.

2. The process according to claim 1, characterized in that the butane is injected in an amount representing up to 200 % by weight of the propane contained in the hydrocarbon charge and, preferably, up to 50 % by weight.

3. The application of the process according to one of claims 1 and 2 for obtaining propane and butane.

**Ansprüche**

1. Verfahren zur Fraktionierung einer Kohlenwasserstoffcharge, welche im wesentlichen aus gesättigten Kohlenwasserstoffen mit 2 bis 4 Kohlenstoffatomen besteht, zur Gewinnung einer propanreichen Fraktion, wobei die Kohlenwasserstoffcharge durch Destillation in wenigstens zwei Fraktionen getrennt wird, nämlich

— eine erste Fraktion A, welche den größten Teil des in der Charge enthaltenen Äthans und eine gewisse Menge an Propan umfaßt und abgekühlt wird, so daß sich eine flüssige Phase, die wieder der Destillation zugeführt wird, und eine Gasphase ergibt, die abgezogen wird, und

— eine zweite Fraktion B, welche den größten Teil des in der Charge enthaltenen Butans und eine gegenüber derjenigen der ersten Fraktion größere Menge an Propan umfaßt, dadurch gekennzeichnet, daß vor der Trennung der flüssigen Phase und der Gasphase der ersten Fraktion A dieser Fraktion Butan zugesetzt wird, welches dann mit der flüssigen Phase wieder der Destillation zugeführt wird, um dort mit der zweiten Fraktion B von der ersten Fraktion A abgetrennt zu werden.

2. Verfahren nach Anspruch 1, dadurch gekennzeichnet, daß das Butan in einer Menge eingebracht wird, welche bis zu 200 Gew.-%, vorzugsweise bis zu 50 Gew.-%, bezogen auf das in der Kohlenwasserstoffcharge enthaltene Propan, ausmachen kann.

3. Anwendung des Verfahrens nach Anspruch 1 oder 2 zur Gewinnung von Propan und Butan.

FIG.1

0 039 289

FIG.2

0 039 289

FIG.3

0 039 289